(19) Europäisches Patentamt
European Patent Office
Office européen des brevets

(11) Veröffentlichungsnummer: **0 235 523**
**A2**

(12) # EUROPÄISCHE PATENTANMELDUNG

(21) Anmeldenummer: **87100649.0**

(51) Int. Cl.[4]: **C07D 319/20** , C07D 319/22

(22) Anmeldetag: **19.01.87**

(30) Priorität: **30.01.86 DE 3602682**

(43) Veröffentlichungstag der Anmeldung:
**09.09.87 Patentblatt 87/37**

(84) Benannte Vertragsstaaten:
**BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Marhold, Albrecht, Dr.**
**Carl-Duisberg-Strasse 329**
**D-5090 Leverkusen 1(DE)**
Erfinder: **Haas, Peter, Dr.**
**Zwengenberger Strasse 43**
**D-5657 Haan 1(DE)**

(54) **Dichlorbenzodioxene.**

(57) Neue Dichlorbenzodioxene der Formel

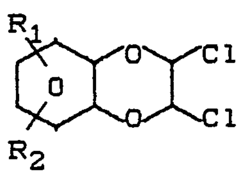

(I),

und ein Verfahren zu ihrer Herstellung aus den entsprechenden Dihydroxybenzodioxenen.

EP 0 235 523 A2

Xerox Copy Centre

## Dichlorbenzodioxene

Die vorliegende Erfindung betrifft neue Dichlorbenzodioxene und eine Verfahren zu ihrer Herstellung.

Es wurden neue Dichlorbenzodioxene gefunden, die durch die Formel (I) gekennzeichnet sind

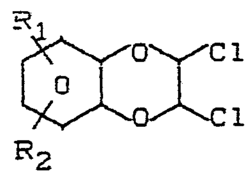

(I),

in der

$R_1$ für Wasserstoff, Alkyl, Alkoxy oder Aroxy und

$R_2$ für Wasserstoff oder zusammen mit $R_1$ für einen Butadienylrest steht.

Soweit $R_1$ Alkyl bedeutet steht es vorzugsweise für $C_1$-bis $C_6$-Alkyl, beispielsweise für Methyl, Ethyl, Propyl, Isopropyl, Butyl, sec.-Butyl, tert.-Butyl, Pentyl oder Hexyl. Soweit $R_1$ Alkoxy bedeutet steht es vorzugsweise für $C_1$-bis $C_6$-Alkoxy, beispielsweise für Methoxy, Ethoxy, Propoxy, Isopropoxy, Butoxy, sec.-Butoxy, tert.-Butoxy, Pentoxy oder Hexoxy. Soweit $R_1$ Aroxy bedeutet steht es vorzugsweise für $C_6$-bis $C_{10}$-Aroxy, beispielsweise für Phenoxy, Methylphenoxy, Dimethylphenoxy, Ethylphenoxy oder Diethylphenoxy.

Besonders bevorzugt steht $R_1$ für Wasserstoff oder Methyl und $R_2$ für Wasserstoff oder gemeinsam mit $R_1$ für einen Butadienylrest.

Ganz besonders bevorzugte Verbindungen der Formel (I) sind 2,3-Dichlor-1,4-benzodioxen, 5-Methyl-2,3-dichlor-1,4-benzodioxen, 2,3-Dichlor-1,4-(5,6-naphthodioxen) und 2,3-Dichlor-1,4-(6,7-naphthodioxen).

Es wurde weiterhin ein Verfahren zur Herstellung von Dichlorbenzodioxenen der Formel (I) gefunden, das dadurch gekennzeichnet ist, daß man die entsprechenden Dihydroxybenzodioxene der Formel (II),

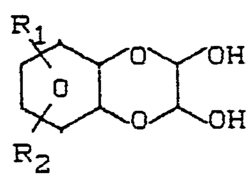

(II)

in der

$R_1$ und $R_2$ die bei Formel (I) angegebene Bedeutung haben, mit Thionylchlorid, Phosphortrichlorid, Phosphoroxychlorid, Phosphorpentachlorid oder Sulfurylchlorid umsetzt.

Die bevorzugten und besonders bevorzugten Bedeutungen von $R_1$ und $R_2$ sind bei Formel (II) ebenso wie bei der Erläuterung der Formel (I) angegeben.

Die Dihydroxybenzodioxene der Formel (II) können beispielsweise aus dem entsprechenden Brenzkatechin und einer wäßrigen Glyoxallösung hergestellt werden (siehe z.B. publizierte Japanische Patentanmeldung 38 613/1971) oder auf dazu analoge Weise. Die Dihydroxybenzodioxene der Formel (II) können auch in ungereinigter, jedoch möglichst wasserfreier Form eingesetzt werden.

Für die erfindungsgemäße Herstellung von Dichlorbenzodioxenen werden stöchiometrisch 2 Mole Chlorierungsmittel aus der Gruppe Thionyl-, Phosphortri-, Phosphoroxy-, Phosphorpenta-und Sulfonylchlorid pro Mol Dihydroxybenzodioxen benötigt. Im allgemeinen wird das Chlorierungsmittel jedoch im Überschuß eingesetzt, beispielsweise 2,01 bis 5 Mol pro Mol Dihydroxybenzodioxen. Das Chlorierungsmittel kann von handelsüblicher Qualität sein. Vorzugsweise wird das Chlorierungsmittel vorgelegt und das jeweilige Dihydroxybenzodioxen dazu gegeben. Von den Chlorierungsmitteln ist Thionylchlorid bevorzugt.

Im allgemeinen ist es vorteilhaft, die erfindungsgemäße Umsetzung in Gegenwart eines Katalysators durchzuführen, beispielsweise in Gegenwart eines tertiären Amins, wie Pyridin oder Dimethylformamid. Der Katalysator kann in beliebigen Mengen angewendet werden, beispielsweise in einer Menge von 0,05 bis 10 Gew.-%, bezogen auf das eingesetzte Chlorierungsmittel.

Für die erfindungsgemäße Umsetzung kommen beispielsweise Temperaturen von Raumtemperatur bis zum Siedepunkt des Chlorierungsmittels oder, falls Phosphorpentachlorid zum Einsatz kommt, bis 120° C in Frage. Beispielsweise sind Temperaturen von 20 bis 100° C geeignet. Bevorzugt sind Temperaturen im Bereich von 50° C bis zum Siedepunkt des Chlorierungsmittels oder, falls Phosphorpentachlorid zum Einsatz kommt, bis 80° C.

Die Reaktion zwischen Dihydroxybenzodioxenen und dem Chlorierungsmittel kann prinzipiell bei verschiedenen Drucken durchgeführt werden. Es ist dabei zu beachten, daß während der Reaktion große Mengen Gase, vor allem Chlorwasserstoff, frei werden, die abzuleiten sind. Vorzugsweise wird bei Normaldruck gearbeitet.

Die erfindungsgemäße Umsetzung ist im allgemeinen dann beendet, wenn das gesamte Dihydroxybenzodioxen eingesetzt worden ist und die Gasentwicklung aufgehört hat. Die hierfür benötigte Zeit hängt im wesentlichen von der Geschwindigkeit des Eintragens des Dihydroxybenzodioxens und diese davon ab, wie schnell das entstehende Gas abgeleitet werden kann. Im allgemeinen ist es vorteilhaft, nach Beendigung der Gasentwicklung noch einige Zeit, z. B. 15 Minuten bis 1 Stunde, die Reaktionsbedingungen aufrecht zu erhalten.

Die Aufarbeitung des nach Beendigung der Reaktion vorliegenden Gemisches kann so erfolgen, daß man zunächst das gegebenenfalls vorhandene überschüssige Chlorierungsmittel entfernt, z.B. durch Destillation, und gegebenenfalls in das erfindungsgemäße Verfahren zurückführt. Der dann verbleibende Rückstand ist ein schon für viele Zwecke ausreichend reines Dichlorbenzodioxen. Falls gewünscht kann man es noch weiter reinigen, z.B. durch Kristallisation oder Destillation, gegebenenfalls unter vermindertem Druck.

Es ist ausgesprochen überraschend, daß in einem stark sauren Milieu unter Entwicklung großer Mengen Chlorwasserstoffgas die erfindungsgemäße Umsetzung überhaupt und sogar mit sehr guten Ausbeuten, meist um 90 % der Theorie, stattfindet, denn gemäß der DE-OS 25 43 886 soll man Dihydroxybenzodioxene bei pH-Werten zwischen 4 und 14, vorzugsweise zwischen 7 und 10 handhaben, da sie im stärker sauren Bereich nicht mehr stabil sind.

Weiterhin ist überraschend, daß das Reaktionsprodukt ganz überwiegend (über 96 %) aus dem jeweiligen trans-Isomeren besteht.

Die neuen Dichlorbenzodioxene können z.B. mit Chlor in Gegenwart von ultraviolettem Licht zu 2,2,3,3-Tetrachlor-1,4-benzodioxenen umgesetzt werden, aus denen sich z.B. durch eine an sich bekannte Fluorierung mit Fluorwasserstoff und gegebenenfalls anschließende Nitrierung und Reduktion Aniline herstellen lassen, aus denen durch Umsetzung mit Acylisocyanaten Verbindungen zugänglich sind, die gemäß den DE-OS en 3 023 328 und 3 023 505 eine überlegene insektizide Wirkung zeigen. Damit können diese Verbindungen mit überlegener insektizider Wirkung aus noch besser zugänglichen Ausgangsstoffen und auf noch einfachere Weise als bisher, sowie in guten Ausbeuten und Reinheiten hergestellt werden.

## Beispiele

### Beispiel 1 (Herstellung des Ausgangsprodukts)

Unter Rühren wurden 200 g 30 %iges, wäßriges Glyoxal und 100 g Brenzkatechin für 3 Stunden auf 80° C erhitzt, dann auf 10° C abgekühlt und die sich bildenden Kristalle abgesaugt. Die Ausbeute an 2,3-Dihydroxy-1,4-benzodioxen mit einem Schmelzpunkt von 123 bis 128° C betrug 138 g.

### Beispiel 2 (Herstellung des Ausgangsprodukts)

Es wurde verfahren wie in Beispiel 1 jedoch wurde anstelle von Brenzkatechin 124 g 3-Methylbrenzkatechin eingesetzt und nach dem Abkühlen das Produkt (5-Methyl-2,3-dihydroxy-1,4-benzodioxen) als Öl von der wäßrigen Phase abgetrennt und getrocknet.

Beispiel 3

In einer Rührapparatur mit Dosiervorrichtung für Festsubstanzen und einer Gasableitung zu einem Wäscher wurden 250 g Thionylchlorid und 2 g Dimethylformamid vorgelegt und auf 65° C erhitzt. Dann wurden 130 g gemäß Beispiel 1 erhaltenes 2,3-Dihydroxy-1,4-benzodioxen portionsweise zudosiert. Es setzte sofort eine hertige Gasentwicklung ein. Wegen der stürmischen Gasentwicklung beanspruchte die Zugabe 5 Stunden. Nachdem 30 Minuten nach Zugabeende bei 70° C gerührt wurde, wurde der Ansatz destilliert. Im Vorlauf wurden bei Normaldruck 50 g Thionylchlorid zurückgewonnen. Bei einem Druck von 0,4 mbar gingen bei 68 bis 100° C 145 g (91 % der Theorie) 2,3-Dichlor-1,4-benzodioxen über. Dieses war gemäß gaschromatographischer Untersuchung 99 % rein und bestand zu über 96 % aus dem trans-Isomeren.

Beispiel 4

500 g Thionylchlorid und 2 ml Dimethylformamid wurden vorgelegt und 206 g gemäß Beispiel 2 erhaltenes 5-Methyl-2,3-dihydroxy-1,4-benzodioxen zugetropft. Es wurde bei 25° C bis zum Ende der Gasentwicklung gerührt und danach noch für 1 Stunde auf 70° C erhitzt. Nach dem Abdestillieren des überschüssigen Thionylchlorids wurde das Produkt durch Destillation gereinigt. Es wurden 115 g 5-Methyl-trans-2,3-dichlor-1,4-benzodioxen mit einem Siedepunkt von 94 bis 96° C bei 0,8 mbar und mit einem Brechungsindex $n_D^{20}$ = 1,5612 erhalten.

**Ansprüche**

1. Dichlorbenzodioxene der Formel

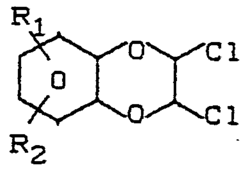

in der
$R_1$ für Wasserstoff, Alkyl, Alkoxy oder Aroxy und
$R_2$ für Wasserstoff oder zusammen mit $R_1$ für einen Butadienylrest steht.

2. Dichlorbenzodioxene gemäß Anspruch 1, dadurch gekennzeichnet, daß $R_1$ für Wasserstoff, $C_1$-bis $C_6$-Alkyl, $C_1$-bis $C_6$-Alkoxy oder $C_6$-bis $C_{10}$-Aroxy und $R_2$ für Wasserstoff oder zusammen mit $R_1$ für einen Butadienylrest steht.

3. Dichlorbenzodioxene gemäß Ansprüchen 1 und 2, dadurch gekennzeichnet, daß $R_1$ für Wasserstoff oder Methyl und $R_2$ für Wasserstoff oder gemeinsam mit $R_1$ für einen Butadienylrest steht.

4. Verfahren zur Herstellung von Dichlorbenzodioxenen der Formel

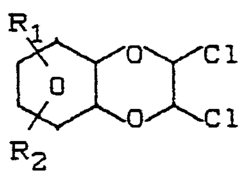

in der
$R_1$ für Wasserstoff, Alkyl, Alkoxy oder Aroxy und
$R_2$ für Wasserstoff oder zusammen mit $R_1$ für einen Butadienylrest steht,
dadurch gekennzeichnet, daß man die entsprechenden Dihydroxybenzodioxene der Formel

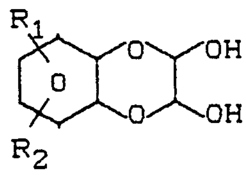

in der

$R_1$ und $R_2$ die oben angegebene Bedeutung haben, mit Thionylchlorid, Phosphortrichlorid, Phosphoroxychlorid, Phosphorpentachlorid oder Sulfurylchlorid umsetzt.

5. Verfahren nach Anspruch 4, dadurch gekennzeichnet, daß man 2 bis 5 Mol Chlorierungsmittel aus der Gruppe Thionylchlorid, Phosphortrichlorid, Phosphoroxychlorid, Phosphorpentachlorid und Sulfurylchlorid pro Mol Dihydroxybenzodioxen einsetzt.

6. Verfahren nach Ansprüchen 4 und 5, dadurch gekennzeichnet, daß man Thionylchlorid einsetzt.

7. Verfahren nach Ansprüchen 4 bis 6, dadurch gekennzeichnet, daß man in Gegenwart eines Katalysators arbeitet.

8. Verfahren nach Anspruch 7, dadurch gekennzeichnet, daß man in Gegenwart eines tertiären Amins arbeitet.

9. Verfahren nach Ansprüchen 4 bis 8, dadurch gekennzeichnet, daß man bei Temperaturen von Raumtemperatur bis zum Siedepunkt des Chlorierungsmittels oder, falls Phosphorpentachlorid zum Einsatz kommt, bis 120° C arbeitet.

10. Verfahren nach Ansprüchen 4 bis 9, dadurch gekennzeichnet, daß man nach Beendigung der Umsetzung gegebenenfalls vorhandenes überschüssiges Chlorierungsmittel durch Destillation entfernt und in das erfindungsgemäße Verfahren zurückführt.